# EUROPÄISCHE PATENTANMELDUNG

(11) **EP 0 609 817 A1**
(43) Veröffentlichungstag der Anmeldung: **10.08.1994**
(21) Anmeldenummer: 94101426.8
(22) Anmeldetag: 31.01.1994
(51) Int. Cl.: A61N 5/06

(54) **Elastische Liegen für Sonnenbänke**

(30) Priorität: 02.02.1993 DE 4302864
(71) Anmelder: IMAB-STIFTUNG, FL-9496 Balzers (LI)
(72) Erfinder: Brück, Gernot K., D-50858 Köln (DE)
(74) Vertreter: Stachow, Ernst-Walther, Prof. Dr.

(57) **Zusammenfassung**

Bei Sonnenbankliegen werden zur gleichmäßigen Bräunung des Körpers häufig auch unterhalb der Liege UV-Licht aussendende Röhren (2) angeordnet.

Die Auflagefläche (5) für den Körper besteht dabei bei bekannten Liegen in der Regel aus einer Plexiglaswanne, da dieses Material eine optimale Transmission für UV-Licht aufweist. Andererseits sind derartige Plexiglaswannen sehr hart und unbequem. Es wird daher vorgeschlagen, einen UV-transparenten und -beständigen sowie elastischen Kunststoff für das Liegenmaterial zu verwenden, der beispielsweise aus einer Fluorpolymerfolie oder einem fluorpolymerbeschichteten Gewebe besteht.

## Beschreibung

### Stand der Technik

Sowohl im professionellen Bräunungsbereich, als auch zur privaten Verwendung finden sich viele Sonnenbänke, die überwiegend mit Quecksilberdampfniederdruckröhren ausgerüstet sind. Der innere Belag dieser Röhren sind spezielle Phosphore, die eine Energieumsetzung in der Weise vornehmen, daß die energiereichere Strahlung dieser Röhren in langwelligere umgesetzt wird. Bei allen professionellen Sonnenbänken in der Niederdrucktechnik und auch bei die teureren Geräten im privaten Bereich, ist nicht nur das Oberteil mil solchen Röhren bestückt, sondern auch das Unterteil, das Bett auf dem man liegt. Hierdurch wird erreicht, das die Bräunung von beiden Seiten in nahezu gleicher Weise erfolgt.

Alle auf dem Markt befindlichen Geräte folgen den gleichen Konstruktionsprinzipien. Im Unterteil befinden sich eine Anzahl von UVRöhren, deren Packungsdichte bei professionellen Geräten maximal ist, sodaß die Röhren mit intergrierten Reflektoren dicht an dicht liegen, ohne jeden Zwischenraum.

Darüber befindet sich eine Plexiglaswanne, auf welcher der Benutzer der Sonnenbank liegt. Da bei den bisher verwendeten Kunststoffen nur das Plexiglas (PMMA) optimale Eigenschaften bezüglich der Transmission von UV-Licht aufwies, wurden alle Sonnenbänke mit einem Plexiglasbett ausgerüstet.

Weiterhin gibt es Hochdruckbänke, bei denen das Oberteil mit einer oder mehreren Reihen aneinanderliegender Einheiten von UV-Strahlungssystem ausgerüstet ist, bestehend aus dem Strahler, einem Reflektor und den vorgeschalteten Filter.

Bisher war es typisch für diese Anlagen, daß das Bett nur aus einer weichen Unterlage bestand, aus der keine Nutzstrahlung kam und somit der Benutzer dieser Anlagen gezwungen war, sich nach einer bestimmten Zeit herum zu drehen, um auch die andere Seite zu besonnen.

### Problem

Da es sich bei Plexiglas um einen sehr harten und spröden Kunststoff handelt, ist der Liegekomfort sehr gering. Nicht nur daß sich das Liegen als ausgesprochen unbequem herausstellt, sondern es bilden sich auch Druckstellen an den Körperteilen, wo die Knochen auf dem Kunststoff aufliegen. Dies ist nicht nur unangenehm im Liegen, sondern führt auch dazu, daß sich diese Stellen nicht bräunen.

Auch ist der Abstand zwischen Röhren und Plexiglaswanne durch die Konstruktion der Sonnenbank fest vorgegeben und kann nicht verändert werden.

Da sich nicht nur die Sonnebank während des Gebrauchs aufheizt, sondern damit auch der Körper, der die breitbandige Infrarotstrahlung absorbiert, setzt verstärkt Transpiration auf.

Bei Hochdruckanlagen ist es schwierig, den richtigen Zeitpunkt zu ermitteln, zu dem man sich drehen muß, um eine gleichmäßige Bräune zu erzielen.

Eine einfache Lösung durch Einsatz von Kunststoff ist aber nicht möglich, da fast alle diese Materialien nicht uv-stabil sind.

### Problemlösung

Um die oben angesprochenen Probleme zu vermeiden, wäre es optimal, wenn die Körperauflage so flexibel wäre, daß sie sich dem Körper anpaßt und damit das Liegen angenehm wird und Druckstellen somit vermieden werden.

Nicht zu letzt bei Hochdruckanlagen würde eine solche elastische Liege das Sonnen vereinfachen, ohne daß auf den bisherigen Komfort der weichen Unterlage verzichtet werden müßte.

### Elastische Liegen für Sonnenbänke

Für die Verwirklichung einer elastischen Liege für Sonnenbänke lassen sich nur Fluorpolymere verwenden, da diese auf jeden Fall und in jeder Hinsicht uv-stabil sind.

Eine einfache effindungsgemäße Vorrichtung ist eine Sonnenbankliege mit einem über den UV-Röhren angebrachtem Spannrahmen, in den eine starke Fluorpolymerfolie eingezogen ist.

Zur Verbesserung des Liegekomforts kann diese einfach eingezogene Folie auch aus einem Folienpaar bestehen, die an den Rändern luftdicht abgeschlossen sind. Hierbei wird dann unterschieden zwischen der den Röhren zugewandte Tragefolie und der dem Körper zugewandte Liegefolie. Zwischen beiden Folien befindet sich ein Luftpolster. Diese Kombination verbindet des Festigkeit einer härteren Tragefolie mit der Elastizität einer weniger festen, dafür aber weicheren Liegefolie.

Höchste Elastizität läßt sich aber nicht durch die Verwendung von Fluorpolymerfolie alleine finden.

Statt einer Fluorpolymerfolie kann auch ein Fluorpolymergewebe eingesetzt werden.

Ein solches Gewebe ist ebenfalls uv-transparent und bezüglich seiner Festigkeit jeder Folie überlegen.

Die noch höhere Elastizität solcher Gewebe ist aber auch mit Nachteilen verbunden. Aufgrund der Maschenzwischenräume kann sich darin Schweiß festsetzten. Eine solche Verwendung widerspricht aber jeder Hygiene. Auch wirkt die den Röhren zugewandte Fläche rauh, sodaß sich Nutzlichtverluste durch Streuung ergeben.

Um ein solches Gewebe dennoch nutzen zu können, wird eine erfindungsgemäße Liege dadurch erreicht, daß das Gewebe beidseitig mit einem gummielastischen Fluorpolymer überzogen wird und dann in den Spannrahmen eingezogen wird.

Die Verbindung eines hochfesten Fluorpolymergewebes mit einem sehr elastischen Fluorpolymerüberzug gibt einem optimale Liegefläche.

Um eine solches beidseitig überzogenes Gewebe herzustellen, wird das Gewebe in eine über die gesamte Breite des Gewebes verlaufende Wanne gezogen, wobei das Gewebe mittels einer ebenfalls mindestens gewebebreiten Rolle durch das Fluorpolymer-Lösungsmittelgemisch gezogen wird.

Hiernach wird das so benetzte Gewebe aus dem Polymer-Lösungsmittelgemisch langsam herausgezogen und oberhalb der Wanne zum Trocknen aufgehängt.

Die Beschichtungsstärke kann eingestellt werden durch die Konzentration des Fluorpolmers im Lösungsmittel und die dadurch entstehende Viskosität des Gemisches, durch die Verdunstungsgeschwindigkeit des Lösungsmittels und durch die Geschwindigkeit, mit der das Gewebe durch das Gemisch gezogen wird.

### Beschreibung

In Figur 1ist eine Aufsicht auf Sonnenbankliegen dargestellt und in den Figuren 2 bis 4 sind jeweils Querschnitte durch die Ebene AB der Sonnenbankliege nach Figur 1 dargestellt.

In den Figuren 5 und 6 ist die Vorrichtung zur Beschichtung des Gewebes dargestellt.

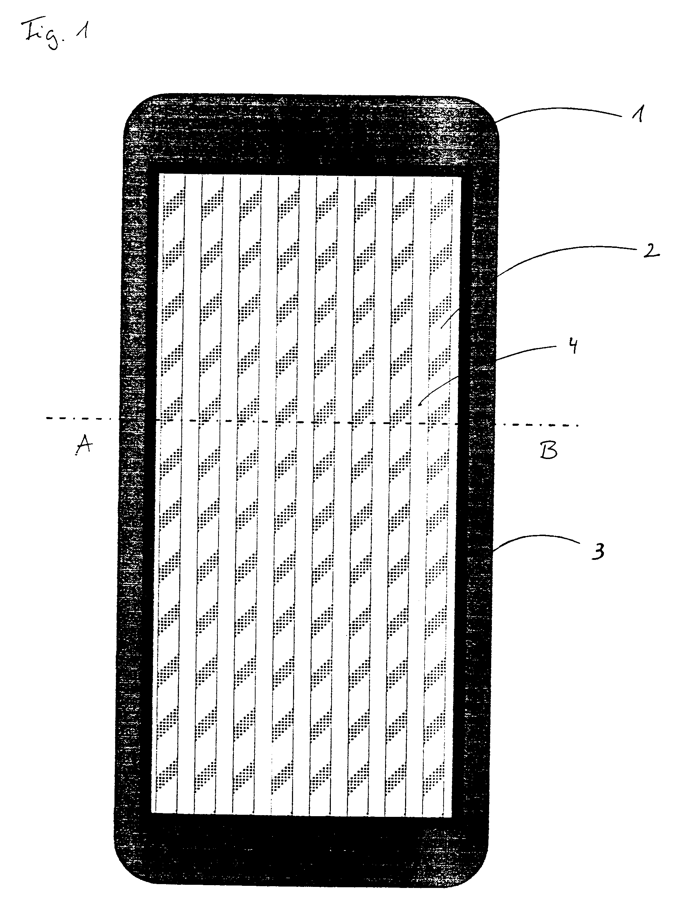

In den Figuren 1 bis 4 sind die folgenden Einzelheiten dargestellt;

Das Gehäuse der Sonnenbankliege 1 in welcher die UV-Röhren 2 eingebaut sind. Ein Spannrahmen 3 nimmt an allen Seiten oder auch nur an zwei gegenüberliegenden Seiten die elastischen Folien oder Gewebe 4 auf.

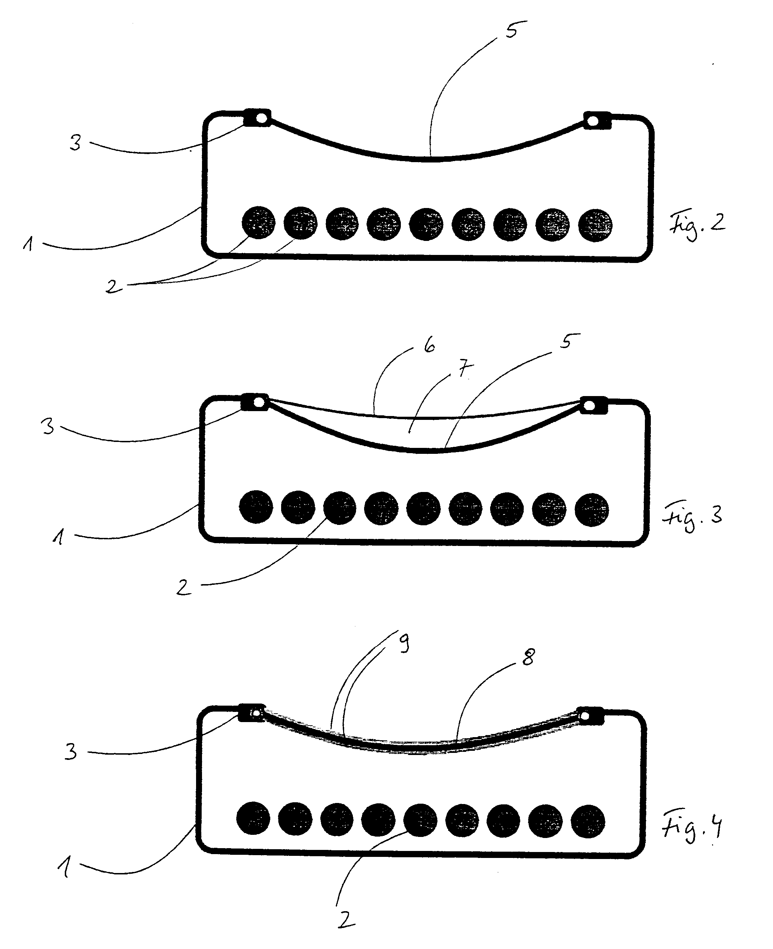

In Figur 2 ist im Spannrahmen 3 eine starke Folie 5 eingezogen, die sich oberhalb der UV-Röhren 2 befindet, wobei der Spannrahmen im Gehäuse 1 verankert ist.

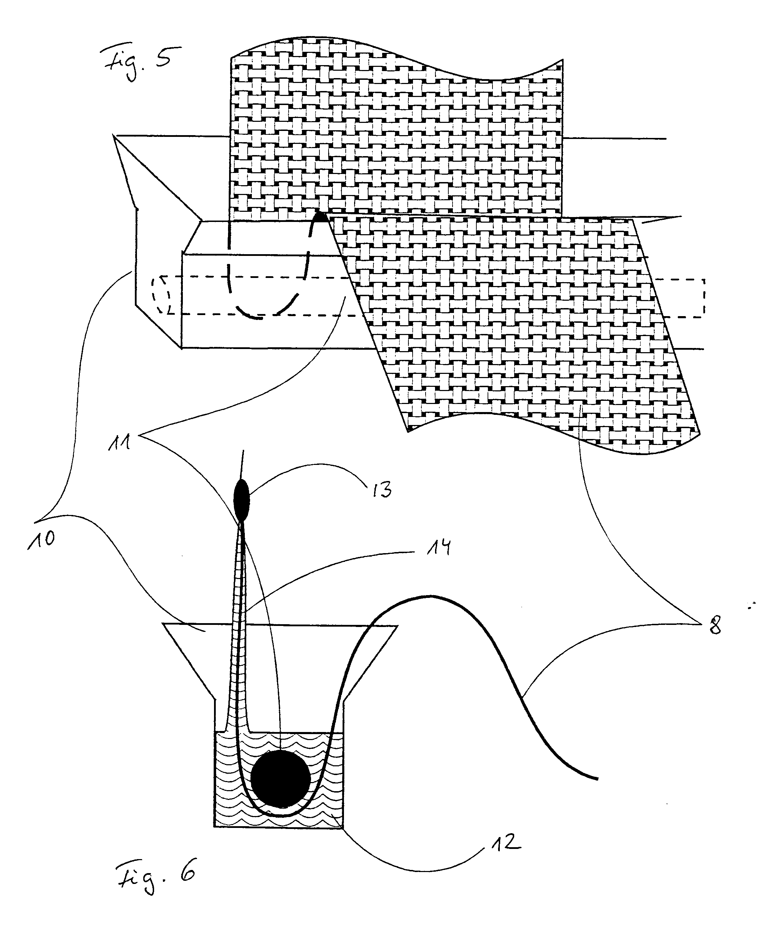

In Figur 3 befindet sich oberhalb der Tragefolie 5 noch eine Liegefolie 6, die durch einen Luftpuffer 7 von der Tragefolie 5 getrennt ist.

In Figur 4 ist ein Gewebe 8 in den Spannrahmen 3 eingezogen, welches beidseitig mit einer Fluorpolymerschicht 9 bedeckt ist.

In den Figuren 5-eine Ansicht- und 6 -ein Querschnitt- sind die folgenden Einzelheiten dargestellt:

In einer Wanne 10 befindet sich eine Rolle 11, die in dem Polymer-Lösungsmittelgemisch 12 eingetaucht angebracht ist.

Das Gewebe 8 wird mittels der Zugvorrichtung 13 in die Wanne 10 gezogen, umläuft die Rolle 11 und nimmt dabei beidseitig das Polymer-Lösungsmittelgemisch auf.

Die nach Durchlauf auf dem Gewebe verbleibende Schicht 14 hängt von den Einstellungsparametern ab.

## Patentansprüche

**Anspruch 1**
Vorrichtung einer Sonnenbankliege dadurch gekennzeichnet, daß die über den Röhren befindliche Liege aus einem elastischen und uv-transparentem Kunststoff gebildet ist.

**Anspruch 2**
Vorrichtung nach Anspruch 1 dadurch gekennzeichnet, daß es sich bei dem Kunststoff um ein Fluorpolymer handelt, welcher nicht nur uv-transparent, sondern auch weitgehend uv-stabil ist

**Anspruch 3**
Vorrichtung nach Ansprüchen 1 und 2 dadurch gekennzeichnet, daß es sich bei dem Kunststoff um eine hochfeste Folie handelt, die in einem Spannrahmen gehalten wird.

**Anspruch 4**
Vorrichtung nach Ansprüchen 1 bis 3 dadurch gekennzeichnet, daß sich oberhalb der Tragefolie noch eine besonders elastische Liegefolie aus Kunststoff befindet und sich dazwischen ein Luftpolster befindet.

**Anspruch 5**
Vorrichtung nach Anspruch 4 dadurch gekennzeichnet, daß es sich bei dem Kunststoff für die Liegefolie ebenfalls um ein Fluorpolymer handelt.

**Anspruch 6**
Vorrichtung nach Ansprüchen 1 und 2 dadurch gekennzeichnet, daß es sich bei dem Liegematerial um ein hochfestes und uv-transparentes Gewebe handelt.

**Anspruch 7**
Vorrichtung nach Ansprüchen 1,2 und 6 dadurch gekennzeichnet, daß das Gewebe in einem Spannrahmen gehalten wird.

**Anspruch 8**
Vorrichtung nach Ansprüchen 1 und 2, sowie 6 und 7 dadurch gekennzeichnet, daß das Gewebe beidseitig mit einem Fluorpolymerkunsstoff beschichtet ist.

**Anspruch 9**
Vorrichtung zur Produktion des Gewebes nach Anspruch 8 dadurch gekennzeichnet, daß das Gewebe mittels einer Zugvorrichtung durch ein Wanne mit einem Fluorpolymer-Lösungsmittelgemisch gezogen wird und anschließend aufgehängt wird.

**Anspruch 10**
Vorrichtung nach Anspruch 9 dadurch gekennzeichnet, daß das Gewebe durch eine im Gemisch befindlichen Rolle geführt wird.

**Anspruch 11**
Verfahren zur Herstellung des Gewebes nach Anspruch 8 mittels der Vorrichtung nach Ansprüchen 9 oder 10 dadurch gekennzeichnet, daß die Beschichtungsstärke durch die Konzentration des Fluorpolymers im Lösungsmittel, die Auswahl des Lösungsmittel und die Bewegungsgeschwindigkeit des Gewebes durch die Vorrichtung nach den Ansprüchen 9 oder 10 eingestellt wird.
